# EUROPÄISCHE PATENTANMELDUNG

(11) **EP 0 538 732 A1**
(43) Veröffentlichungstag der Anmeldung: **28.04.1993**
(21) Anmeldenummer: 92117598.0
(22) Anmeldetag: 15.10.1992
(51) Int. Cl.: C22B 3/38, B01J 31/40, C22B 11/00

(54) **Verfahren zur Rückgewinnung von Rhodium aus den Reaktionsprodukten der Oxosynthese**

(30) Priorität: 24.10.1991 DE 4135049
(71) Anmelder: HOECHST AKTIENGESELLSCHAFT, D-65926 Frankfurt am Main (DE)
(72) Erfinder: Förster, Ingrid, W-4330 Mülheim/Ruhr (DE); Mathieu, Klaus, W-4200 Oberhausen (DE)

(57) **Zusammenfassung**

Die Erfindung betrifft ein Verfahren zur Abtrennung und Rückgewinnng von Rhodium aus Reaktionsprodukten der Oxosynthese, die unter Verwendung des Edelmetalls ohne zusätzliche Komplexbildner als Katalysator erhalten wurden. Die Abtrennung des Edelmetalls erfolgt mit der wäßrigen Lösung eines organischen Phosphins aus dem unter Druck stehenden Reaktionsprodukt in Form einer wasserlöslichen, das organische Phosphin als Ligand enthaltenden Rhodiumcarbonylverbindung.

## Beschreibung

Die Erfindung betrifft ein Verfahren zur Abtrennung und Rückgewinnung von Rhodium aus solchen Reaktionsprodukten der Oxosynthese, die unter Verwendung des Edelmetalls ohne zusätzliche Komplexbildner als Katalysator erhalten wurden.

Die Herstellung von Aldehyden und Alkoholen durch Anlagerung von Kohlenmonoxid und Wasserstoff an olefinische Doppelbindungen (Hydroformylierung) ist bekannt. Die Reaktion wird durch Metalle der VIII. Gruppe des Periodensystems der Elemente oder deren Verbindungen katalysiert, die unter den Reaktionsbedingungen Carbonyle oder Hydrocarbonyle bilden. Während früher fast ausschließlich Kobalt und Kobaltverbindungen als Katalysatoren eingesetzt wurden, finden heute in zunehmendem Maße Rhodiumkatalysatoren Anwendung, obgleich Rhodium um ein Vielfaches teurer als Kobalt ist. Rhodium gelangt dabei allein oder in Kombination mit Komplexbildnern, z. B. organischen Phosphinen oder Phosphiten zum Einsatz. Während die Oxosynthese mit Rhodium als Katalysator Reaktionsdrücke von 25 bis 30 MPa erfordert, genügen bei Einsatz von Rhodium in Kombination mit Komplexbildnern Drücke von 1 bis 5 MPa.

Für Rhodiumkatalysatoren ergeben sich in vielen Fällen deutliche Vorteile. Sie besitzen höhere Aktivität und Selektivität und ermöglichen überdies einen problemfreien Betrieb der Produktionsanlage, insbesondere hinsichtlich der Durchführung der Synthese und der Ausbringung der Produkte aus dem Reaktor. Schließlich kann das klassische Oxoverfahren auf Basis von Kobaltkatalysatoren unter Verwendung der vorhandenen Apparateteile in vielen Fällen mit nur geringen Investitionen auf Rhodiumkatalysatoren umgestellt werden.

Erhebliche Schwierigkeiten bereitet es jedoch, das ohne Komplexbildner als Katalysator eingesetzte Rhodium unter Ausschluß von Verlusten aus dem Reaktionsprodukt abzutrennen und wiederzugewinnen.

Entsprechend dem Stand der Technik wird das Reaktionsprodukt der Oxosynthese üblicherweise in mindestens zwei Stufen entspannt. Zunächst vermindert man, um gelöstes Synthesegas abzutrennen, den Druck von Synthesedruck, d.h. etwa 25 bis 30 MPa, auf 1,5 bis 2,5 MPa, anschließend reduziert man, gegebenenfalls über Zwischenstufen, auf Normaldruck. Vor der Reinigung des rohen Reaktionsproduktes, z.B. durch Destillation oder seiner Weiterverarbeitung zu Folgeprodukten, müssen die in ihm homogen gelösten Rhodiumverbindungen, die in einer Konzentration von nur wenigen ppm vorliegen, abgetrennt werden. Dabei ist zu beachten, daß das Rhodium bei der Entspannung teilweise in metallische Form übergeht oder mehrkernige Carbonyle bildet. In beiden Fällen kommt es dann zur Ausbildung eines heterogenen Systems, das aus der flüssigen organischen Phase und der festen, Rhodium oder Rhodiumverbindungen enthaltenden Phase, besteht.

Nach dem Verfahren der DE-A1 33 47 406 wird das Rhodium mit Hilfe komplexbildender Reagenzien aus dem Rohprodukt, also dem nach Entspannen und gegebenenfalls Abkühlen anfallenden Reaktionsgemisch unter Normaldruck abgetrennt und wiedergewonnen. Vorzugsweise setzt man als Komplexbildner Sulfonate oder Carboxylate organischer Phosphine ein, die mit dem Rhodium wasserlösliche Komplexverbindungen ergeben. Daher kann das Edelmetall mit wäßrigen Lösungen der Phosphine extrahiert werden. Das Rhodium geht hierbei in die wäßrige Phase über, die sich durch einfaches Dekantieren vom organischen Produktgemisch abtrennen läßt. Durch Kreislaufführung der Lösung des Komplexbildners können hohe Rhodiumkonzentrationen in der wäßrigen Phase erreicht werden.

Um die Extraktion des Rhodiums aus der organischen Phase und seinen Übergang in die wäßrige Phase zu beschleunigen und zu vervollständigen, setzt man nach der DE-A1 34 11 034 der wäßrigen Lösung des Komplexbildners einen Lösungsvermittler zu. Seine Wirkung besteht vor allem darin, daß er die physikalischen Eigenschaften der Grenzflächen zwischen den beiden flüssigen Phasen verändert und dadurch den Übergang des wäßrigen Extraktionsmittels in die Produktphase und des Rhodiums aus der Produktphase in die wäßrige Komplexbildnerphase beschleunigt. Durch Mitverwendung eines Lösungsvermittlers wird die Extraktion vereinfacht und der apparative Aufwand verringert.

Eine Weiterentwicklung des vorstehenden Verfahrens ist in der DE-A1 34 43 474 beschrieben. Es verwendet zur Extraktion von Rhodium aus den Produkten der Oxosynthese Tetraorganylammoniumsalze sulfonierter Triarylphosphine, die sowohl komplexbildend wirken als auch lösungsvermittelnde Eigenschaften besitzen.

Die bekannten Prozesse haben sich in der Praxis ausgezeichnet bewährt. Sie ermöglichen es im technischen Betrieb bis zu etwa 95 % des ursprünglich eingesetzten Rhodiums aus den Oxoreaktionsprodukten abzutrennen und wieder nutzbar zu machen. Im Hinblick auf den hohen Preis des Edelmetalls besteht jedoch Interesse daran, die Wiedergewinnung des Rhodiums weiter zu verbessern.

Diese Aufgaben löst die Erfindung mit Hilfe eines Verfahrens zur Abtrennung und Wiedergewinnung von Rhodium aus den Produkten der Oxosynthese durch Extraktion mit der wäßrigen Lösung eines komplexbildenden organischen Phosphins. Es ist dadurch gekennzeichnet, daß das Rhodium aus dem unter Druck stehenden Reaktionsprodukt als wasserlösliche phosphinsubstituierte Rhodiumcarbonylverbindung extrahiert wird.

Überraschenderweise kann durch den erfindungsgemäßen Prozess der Anteil des zurückgewonnenen Rhodiums, bezogen auf eingesetztes Metall, verglichen mit dem Stand der Technik, weiter erhöht werden.

Ein wesentliches Merkmal des neuen Verfahrens ist es, das im Produkt der Oxosynthese gelöste Rhodium unter erhöhtem Druck zu extrahieren. Diese Maßnahme verhindert, daß sich die katalytisch wirksame Rhodiumcarbonylverbindung zersetzt und dadurch der unmittelbaren Reaktion mit dem wasserlöslichen Phosphin entzieht. Nach der von den Fachleuten überwiegend vertretenen Meinung ist Rhodiumhydridocarbonyl die katalytisch wirksame Verbindung bei der durch Rhodium katalysierten Hydroformylierung (vgl. z.B. Greenwood, Earnshaw, Chemistry of the Elements, 1984, S. 1317-1319). Daher sind bei Durchführung des erfindungsgemäßen Verfahrens im Reaktionsprodukt im Anschluß an die Synthese Druckbedingungen aufrechtzuerhalten, unter denen bei der gewählten Temperatur das Rhodiumhydridocarbonyl existenzfähig ist. Bewährt hat sich die Einhaltung von Temperaturen zwischen 0 und 200°C, bevorzugt 20 bis 130°C. Nach einer speziellen Ausführungsform der Erfindung führt man die Extraktion mit dem wasserlöslichen organischen Phosphin unter den Bedingungen der Hydroformylierung durch, d.h. in Anwesenheit von Synthesegas und bei Synthesetemperatur und -druck. Dieses Vorgehen steht im Gegensatz zu den bekannten Aufarbeitungsprozessen, bei denen das Reaktionsprodukt zunächst entspannt und entgast wird, bevor die Rhodiumabtrennung erfolgt.

Zur Komplexbildung mit Rhodium befähigte wasserlösliche organische Phosphine sind insbesondere Verbindungen der allgemeinen Formel 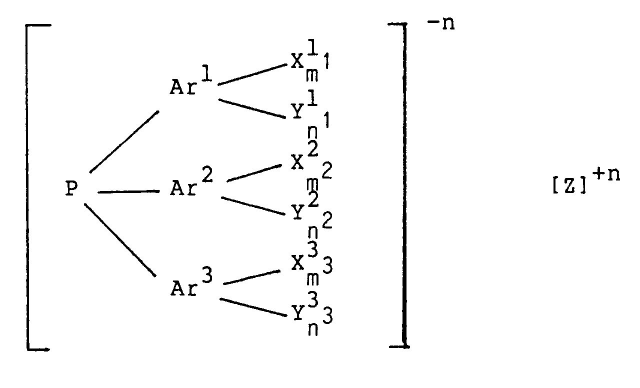
wobei Ar¹, Ar², Ar³ jeweils ein Benzol- oder Naphthalinrest, Y¹, Y², Y³ jeweils eine geradkettige oder verzweigte Alkylgruppe mit 1 bis 4 Kohlenstoffatomen, eine Alkoxygruppe, ein Halogenatom, die OH-, CN-, NO₂- oder R¹R²N-Gruppe in der R¹ und R² jeweils für eine geradkettige oder verzweigte Alkylgruppe mit 1 bis 4 Kohlenstoffatomen stehen, bedeuten, X¹, X², X³ jeweils ein Sulfonat-(-SO₃⁻) oder Carboxylat-(-COO⁻) Rest sind, m¹, m², m³ gleiche oder verschiedene ganze Zahlen von 0 bis 3 sind und mindestens eine Zahl m¹, m², m³ gleich oder größer 1 ist, n¹, n², n³ gleiche oder verschiedene ganze Zahlen von 0 bis 5 sind, n eine ganze Zahl von 1 bis 9 ist und Z für Alkalimetall-, Ammoniumionen oder für das Äquivalent eines Erdalkalimetalls oder des Zinks steht.

Als Extraktionsmittel im Rahmen der neuen Arbeitsweise besonders geeignet sind Phosphine der oben wiedergegebenen allgemeinen Formel in der Ar¹, Ar², Ar³ jeweils einen nicht oder durch einen Sulfonatrest substituierten Benzolrest bedeuten, mit der Maßgabe, daß mindestens ein Benzolrest durch einen Sulfonatrest substituiert ist, also X¹, X² und/oder X³ für -SO₃⁻ steht, m¹, m², m³ 0 oder 1 und die Summe von m¹, m² und m³ 1, 2 oder 3 ist und weiterhin n¹, n² und n³ jeweils Null sind. Kationen Z⁺ der wasserlöslichen organischen Phosphine sind vorzugsweise Alkalimetallionen, insbesondere Natriumionen. Es ist nicht erforderlich, die wasserlöslichen organischen Phosphine als einheitliche Verbindungen einzusetzen. Auch Gemische verschiedener Phosphine können im erfindungsgemäßen Verfahren angewandt werden. Beispiele für Phosphine, die sich zur Extraktion von Rhodium bewährt haben, sind das Tri-Na-phenyltrisulfophenylphosphin (abgekürzt TPPTS genannt), das Di-Na-phenyldisulfophenylphosphin (abgekürzt TPPDS genannt) und das Mono-Na-diphenylsulfophenylphosphin (abgekürzt TPPMS genannt). Die Verbindungen werden einzeln oder, bevorzugt, als Gemisch verwendet.

Um das Eindringen der wäßrigen (Extraktions-) Phase in das organische Reaktionsprodukt zu erleichtern und dadurch Geschwindigkeit und Vollständigkeit der Extraktion zu erhöhen, kann man die Extraktion in Gegenwart handelsüblicher Lösungsvermittler (Phasentransferreagentien) vornehmen. Geeignet sind sowohl anionische Reagentien, wie Laurin-, Myristin- oder Stearinsäure, als auch kationische Reagentien, nämlich Amine, wie Octadecyldiethylamin, Octadecylethanolamin und neutrale Reagentien, z.B. Addukte des Ethylenoxids an höhermolekulare Alkohole, an Phenole und an Fettsäuren.

Auf Rhodium bezogen werden die komplexbildenden Phosphine im molaren Überschuß angewendet. Da man sie zur erneuten Extraktion rezirkulieren kann, ist die Höhe des Überschusses unkritisch, jedoch sollen je mol Rhodium mindestens 5 mol komplexbildendes Phosphin vorhanden sein. Besonders bewährt hat es sich, je mol Rhodium 60 bis 100 mol, bei Rückführung sogar bis 500 mol komplexbildendes Phosphin zu verwenden.

Üblicherweise wird das komplexbildende Phosphin in Form einer Lösung eingesetzt. Hierbei ist darauf zu achten, daß es ebenso wie der entstehende Rhodiumkomplex, im Reaktionsprodukt weitgehend unlöslich, dagegen im Lösungsmittel für das komplexbildende Reagenz gut löslich ist. Selbstverständlich darf auch das Lösungsmittel mit dem Reaktionsprodukt nicht oder nur sehr wenig mischbar sein.

Bei Erfüllung dieser Bedingungen bildet sich ein Zweiphasensystem aus, das aus dem Reaktionsprodukt und der Lösung des komplexbildenden Phosphins bzw. der entstandenen Rhodium-Komplexverbindung besteht. Der Komplexbildner wirkt als Extraktionsmittel, d.h. im Anfangszustand befindet sich das Rhodium im Reaktionsprodukt gelöst, im Endzustand in der Lösung des Komplexbildners. Die Trennung von Reaktionsprodukt und Rhodium enthaltender Lösung erfolgt nach den hierfür üblichen Grundoperationen der chemischen Verfahrenstechnik z.B. durch Dekantieren oder Zentrifugieren.

Das bevorzugte Lösungsmittel für das komplexbildende Reagenz und den Rhodiumkomplex ist Wasser. Es können auch Gemische von Wasser mit anderen Lösungsmitteln, z.B. niederen Alkoholen wie Methanol, verwendet werden, sofern sichergestellt ist, daß Oxoreaktionsprodukt und Lösungsmittel sich miteinander nicht mischen und die Löslichkeit des Rhodium-Phosphin-Komplexes in der Wasserphase nicht beeinträchtigt wird.

Die Konzentration des komplexbildenden Reagenzes in der Lösung ist in weiten Grenzen variabel. Sie hängt insbesondere davon ab, in welchem Maße das Rhodium angereichert werden soll. Dementsprechend können nicht nur gesättigte, sondern sogar stark verdünnte Lösungen eingesetzt werden. In der Regel verwendet man Lösungen, die 0,5 bis 50 Gew.-% und vorzugsweise 5 bis 35 Gew.-% (jeweils bezogen auf die Lösung) des Komplexbildners enthalten.

Sofern der Komplexbildner unter den Extraktionsbedingungen flüssig und im Hydroformylierungsprodukt unlöslich oder schwerlöslich und weiterhin der Rhodiumkomplex im komplexbildenden Phosphin löslich ist, kann auf die Mitverwendung eines Lösungsmittels auch verzichtet, also der reine Komplexbildner eingesetzt werden.

Der neue Prozeß läßt sich sowohl absatzweise als auch kontinuierlich durchführen.

Die Weiterbehandlung oder Weiterverwendung der das abgetrennte Rhodium enthaltenden Phase richtet sich nach den jeweiligen Gegebenheiten. So kann das Rhodium in bekannter Weise, z.B. durch Überführung in das Salz einer höheren Carbonsäure, abgetrennt und wiederum als Katalysator eingesetzt werden. Es ist aber auch möglich, die aus Lösungsmittel, Rhodium und Komplexbildner bestehende Phase unmittelbar als Katalysatorsystem zu verwenden.

Das erfindungsgemäße Verfahren ist zur Abtrennung und Rückgewinnung von Rhodium aus den verschiedensten Produkten der Oxosynthese geeignet. Es kann nicht nur mit Erfolg bei Rohprodukten angewandt werden, die durch Hydroformylierung geradkettiger oder verzweigter acyclischer, olefinischer Kohlenwasserstoffe, insbesondere solcher mit 2 bis 20 Kohlenstoffatomen entstehen. Auch bei der Abtrennung von Rhodium aus Produkten der Hydroformylierung anderer olefinisch ungesättigter Verbindungen, z.B. ungesättigter Alkohole, Aldehye, Carbonsäuren, ferner Diolefine, cyclischer Olefine wie Dicyclopentadien, hat es sich ausgezeichent bewährt.

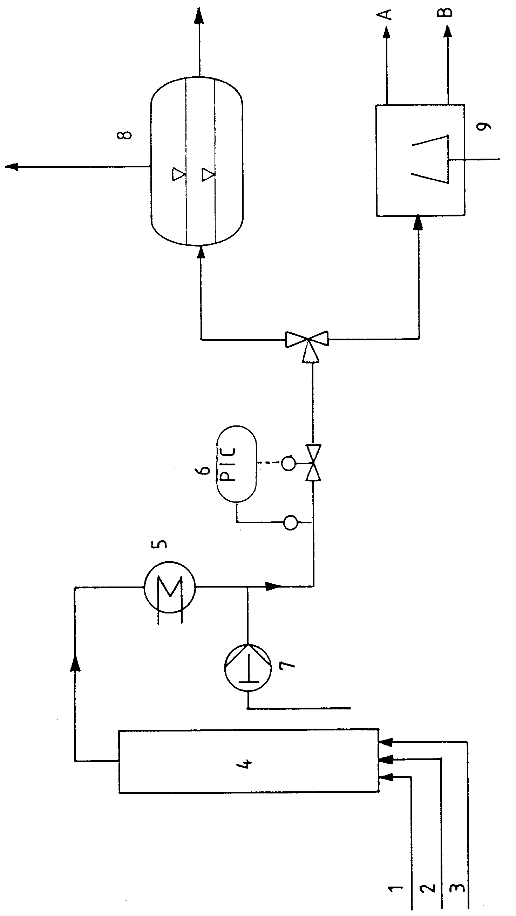

Eine technische Ausführungsform der neuen Arbeitsweise ist in der beigefügten Abbildung dargestellt. Selbstverständlich läßt sich der erfindungsgemäße Prozeß auch in anderen Verfahrensvarianten realisieren.

Einem Reaktor 4 werden über Leitungen 1, 2 und 3 Synthesegas, Olefin und in einer organischen Phase homogen gelöster Rhodiumkatalysator zugeführt. Das in einem Wärmetauscher 5 gegebenenfalls abgekühlte Produkt wird über ein Ventil 6 abgezogen, wobei mit Synthesegas ein ausreichender Druck aufrechterhalten wird, um eine Zersetzung des Rhodiumhydrocarbonyls zu verhindern. Dem Produktstrom wird in einem Rohrleitungsabschnitt über eine Pumpe 7 eine den Komplexbildner enthaltende wäßrige Phase zugemischt. Auf diese Weise erreicht man eine intensive Durchmischung der Phasen und eine nahezu vollständige Extraktion des Rhodiums. Organische und wäßrige Phase werden im Trenngefäß 8 oder in der Zentrifuge 9 getrennt.

In den folgenden Beispielen wird die Erfindung näher beschrieben. Sie ist jedoch nicht auf diese speziellen Ausführungsformen beschränkt. Alle Konzentrationsangaben erfolgen in Gewichtsprozent (Gew.-%).

### Beispiel 1

In einem mit Magnetrührer ausgerüsteten Autoklaven wurde Dicyclopentadien bei 130°C und einem Synthesegasdruck (CO : H₂ = 1 : 1) von 27 MPa in Gegenwart von 60 Gew.-ppm Rhodium (bezogen auf Dicyclopentadien) als Katalysator hydroformyliert.

Nach Beendigung der Reaktion wurde der Druck auf 25 MPa gesenkt, bei 130°C eine wäßrige TPPTS-Lösung in einer Menge zugepumpt, daß das P(III)/Rh-Molverhältnis 100 : 1 betrug und etwa 2 bis 3 min gerührt. Anschließend wurde das Reaktionsgemisch in einen Rührkolben mit Bodenablaß überführt und unter Rühren auf 60°C erwärmt. Nach Beendigung des Rührens setzte die Phasentrennung sofort ein, sie war nach etwa 30 min abgeschlossen. Die Analyse der Wasserphase ergeb, daß 94 bis 96 % des zur Hydroformylierung eingesetzten Rhodiums extrahiert worden waren. Durch eine Nachwäsche der organischen Phase konnte der Anteil des zurückgewonnenen Rhodiums auf 97 bis 98 % (bezogen auf die in die Hydroformylierung eingesetzte Rhodiummenge) erhöht werden, entsprechend einer Rhodium-Restkonzentration in der organischen Phase von 0,7 bis 0,9 Gew.-ppm. Alternativ können die Phasen durch Zentrifugieren bei Raumtemperatur getrennt werden; in diesem Fall ist eine Nachwäsche nicht erforderlich.

### Beispiele 2 bis 4

Beispiel 1 wurde unter geänderten Druck- und Temperaturbedingungen wiederholt. Reaktionsbedingungen und Extraktionsergebnisse sind in der nachfolgenden Tabelle zusammengestellt.

**Tabelle**

| Temperatur (°C) | Druck (MPa) | Rh-Rückgewinnung* |
|---|---|---|
| 80 | 27,0 | 98,6 |
| 80 | 5,0 | 97,0 |
| 130 | 5,0 | 97,8 |

| | | |
|---|---|---|
| * in %, bezogen auf in die Hydroformylierung eingesetztes Rhodium | | |

## Patentansprüche

1. Verfahren zur Abtrennung und Wiedergewinnung von Rhodium aus den Produkten der Oxosynthese durch Extraktion mit der wäßrigen Lösung eines komplexbildenden organischen Phosphins, dadurch gekennzeichnet, daß das Rhodium aus dem unter Druck stehenden Reaktionsprodukt als wasserlösliche phosphinsubstituierte Rhodiumcarbonylverbindung extrahiert wird.

2. Verfahren nach Anspruch 1, dadurch gekennzeichnet, daß die komplexbildenden organischen Phosphine Verbindungen der allgemeinen Formel 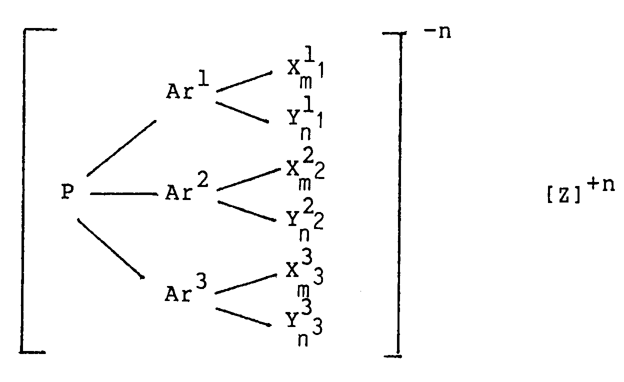 wobei Ar¹, Ar², Ar³ jeweils ein Benzol- oder Naphthalinrest, Y¹, Y², Y³ jeweils eine geradkettige oder verzweigte Alkylgruppe mit 1 bis 4 Kohlenstoffatomen, eine Alkoxygruppe, ein Halogenatom, die OH-, CN-, NO₂- oder R¹R²N-Gruppe in der R¹ und R² jeweils für eine geradkettige oder verzweigte Alkylgruppe mit 1 bis 4 Kohlenstoffatomen stehen, bedeuten, X¹, X², X³ jeweils ein Sulfonat-(-SO₃⁻) oder Carboxylat-(-COO⁻) Rest sind, m¹, m², m³ gleiche oder verschiedene ganze Zahlen von 0 bis 3 sind und mindestens eine Zahl m¹, m², m³ gleich oder größer 1 ist, n¹, n², n³ gleiche oder verschiedene ganze Zahlen von 0 bis 5 sind, n eine ganze Zahl von 1 bis 9 ist und Z für Alkali-metall-, Ammoniumionen oder für das Äquivalent eines Erdalkalimetalls oder des Zinks steht, sind.

3. Verfahren nach Anspruch 2, dadurch gekennzeichnet, daß Ar¹, Ar², Ar³ jeweils einen Benzolrest bedeutet, X¹, X² und/oder X³ für den Sulfonatrest steht, die Summe von m¹, m² und m³ 1 bis 3 ist und n¹, n² und n³ jeweils Null sind.

4. Verfahren nach Anspruch 2 oder 3, dadurch gekennzeichnet, daß das Kation Z⁺ⁿ vorzugsweise ein Alkalimetallion, insbesondere ein Natriumion ist.

5. Verfahren nach einem oder mehreren der Ansprüche 1 bis 4, dadurch gekennzeichnet, daß das Rhodium bei Temperaturen zwischen 0 und 200°C, vorzugsweise 20 und 130°C, extrahiert wird.

6. Verfahren nach einem oder mehreren der Ansprüche 1 bis 4, dadurch gekennzeichnet, daß das Rhodium unter den Temperatur- und Druckbedingungen der Hydroformylierung extrahiert wird.

7. Verfahren nach einem oder mehreren der Ansprüche 1 bis 6, dadurch gekennzeichnet, daß je mol Rhodium mindestens 5, vorzugsweise 60 bis 100 mol wasserlösliches organisches Phosphin verwendet werden.

8. Verfahren nach einem oder mehreren der Ansprüche 1 bis 7, dadurch gekennzeichnet, daß die Konzentration des wasserlöslichen organischen Phosphins in der wäßrigen Lösung 0,5 bis 50 Gew.-%, vorzugsweise 5 bis 35 Gew.-% beträgt.

9. Verfahren nach einem oder mehreren der Ansprüche 1 bis 8, dadurch gekennzeichnet, daß die Extraktion des Rhodiums in Gegenwart eines Lösungvermittlers erfolgt.
